# EUROPEAN PATENT APPLICATION

(11) **EP 4 151 211 A1**
(43) Date of publication of application: **22.03.2023**
(21) Application number: 21804702.5
(22) Date of filing: 14.05.2021
(51) Int. Cl.: A61K 31/4353, A61K 9/08, A61K 9/14, A61K 9/72, A61P 31/14

(54) **NOVEL INHALANT**

(30) Priority: 14.05.2020 JP 2020085471
(71) Applicant: KOWA COMPANY, LTD., Naka-ku Nagoya-shi Aichi 460-8625 (JP)
(72) Inventor: OKUMURA, Mutsuo, Tokyo 103-8433 (JP); MURAKAMI, Seiichi, Higashimurayama-shi, Tokyo 189-0022 (JP)
(74) Representative: Hartz, Nikolai
(86) International application number: PCT/JP2021/018328
(87) International publication number: WO 2021/230340

(57) **Abstract**

Provided is a novel inhalant as a therapeutic agent for COVID-19. The present invention relates to an inhalant containing cepharanthine.

## Description

### Technical Field

The present invention relates to an inhalant containing cepharanthine.

### Background Art

COVID-19 (Japanese name: novel coronavirus infectious disease) is an infectious disease caused by a 2019 novel coronavirus (SARS-CoV-2). COVID-19 is an infectious disease, the outbreak of which was confirmed in Wuhan, People's Republic of China in November, 2019 and was reported to WHO in December of that year, and after this, the infection spread worldwide. The symptoms thereof begin with fever, dry cough, fatigue, spitting, shortness of breath, sore throat, headache, muscle ache, arthralgia, dysosmia, dysgeusia, and the like, and in severe cases, pneumonia becomes severe, leading to respiratory failure and to the outcome of death.

There are still unclear aspects, such as the infectability and the aggravation rate when infected, besides, effective treatments are still being searched since the virus is a new type. This has been perturbing people around the world.

A large number of existing drugs have been screened so far, and cepharanthine has recently been expected as a therapeutic agent for COVID-19 (Non Patent Literature 1).

### Citation List

### Non Patent Literature

Non Patent Literature 1: https://www.tus.ac.jp/mediarelations/archive/20200422_983 7.html

### Summary of the Invention

### Technical Problem

An object of the present invention is to provide a novel medication for prevention and/or treatment of COVID-19, which is a disease based on SARS-CoV-2 infection.

### Solution to Problem

The above-mentioned cepharanthine is presently used as an oral administration preparation of a powder or a tablet, or an injection preparation. The present inventors conceived to apply cepharanthine, which is considered as an effective drug for inhibition of infection by SARS-CoV-2, to a site that is most vulnerable to infection by SARS-CoV-2. As a result, it was found that the purpose can be achieved by forming cepharanthine into an inhalant that can be directly applied to the lower respiratory tract, and it was further found that an inhalant that can efficiently supply cepharanthine directly to the lower respiratory tract is obtained by adjusting the particle diameter of cepharanthine to 0.5 to 10 µm, and the present invention was accomplished.

That is, the present invention provides an inhalant comprising cepharanthine. In addition, cepharanthine efficiently reaches the lower respiratory tract by adjusting the particle diameter of cepharanthine to 0.5 to 10 µm as the aerodynamic particle diameter and the aerodynamic mass median diameter of cepharanthine to 0.5 to 10 µm.

### Advantageous Effects of the Invention

According to the inhalant of the present invention, cepharanthine, which is considered as an effective drug for inhibition of infection by SARS-CoV-2, can be directly supplied to the lower respiratory tract.

### Description of Embodiment

The inhalant of the present invention is a preparation that allows cepharanthine to be inhaled as aerosol and to be applied to the lower respiratory tract.

The cepharanthine used in the present invention is a chemical substance, the chemical name of which is 6',12'-dimethoxy-2,2'-dimethyl-6,7-[methylenebis(oxy)]oxyacanthan, and is one of alkaloids that can be extracted from tamasaki-tsuzurafuji (*Stephania cepharantha* Hayata), kohto-tsuzurafuji (*Stephania sasakii* Hayata), snake vine (*Stephania japonica*)*,* and the like.

Cepharanthine is used for treatment of radiation-induced leukopenia, alopecia areata/alopecia pityriasis, secretory otitis media, and pit viper bite. When cepharanthine is used as an inhalant as in the present invention, in addition to these indications, the inhalant is particularly useful as a SARS-CoV-2 infection inhibitor and a COVID-19 prophylactic and/or therapeutic agent.

In the present invention, cepharanthine can be either chemically synthesized or extracted from tamasaki-tsuzurafuji, kohto-tsuzurafuji, snake vine, and the like. When cepharanthine is extracted from tamasaki-tsuzurafuji, it is possible to use a tamasaki-tsuzurafuji extract containing, in addition to cepharanthine, an alkaloid other than cepharanthine, such as isotetrandrine, cycleanine, and berbamine. There are commercial products of cepharanthine and the tamasaki-tsuzurafuji extract, and they can be purchased and used. Alternatively, the tamasaki-tsuzurafuji extract can be prepared from tamasaki-tsuzurafuji using a known method.

In order to apply the cepharanthine used in the present invention to the lower respiratory tract (the trachea, bronchi, and lungs), from the viewpoint of reachability of cepharanthine to the lower respiratory tract, the particle diameter is preferably adjusted to 0.5 to 10 µm, more preferably 1 to 8 µm, and further more preferably 2 to 8 µm. Specifically, the aerodynamic mass median diameter of cepharanthine is preferably adjusted to 0.5 to 10 µm, more preferably 1 to 8 µm, and further more preferably 2 to 8 µm. Here, for a powder, the particle diameter of cepharanthine is the particle diameter of the cepharanthine-containing powder; and for a liquid, the diameter is the particle diameter of the cepharanthine-containing spray droplet. This particle diameter in the case of a powder can be adjusted by pulverization, sieving, and the like during the preparation of the powder. For a spray droplet, the diameter may be appropriately adjusted according to the configuration of the nebulizer (such as a jet type, an ultrasonic type, and a mesh type) used when the liquid is inhaled.

Examples of the form of the inhalant of the present invention include an inhalation powder, an inhalation liquid, and an inhalation aerosol but it is not particularly limited. When the inhalant of the present invention is used, a tool or device suitable for inhalation administration is used, or a container combined with a tool for inhalation may be filled with the inhalant.

The inhalation powder is a preparation for inhalation as an aerosol of a cepharanthine-containing powder prepared such that the inhalation amount may be constant. The cepharanthine-containing powder may be prepared as a powder having a particle diameter of preferably 0.5 to 10 µm, more preferably 1 to 8 µm, and further more preferably 2 to 8 µm, specifically, may be prepared as a powder having an aerodynamic mass median diameter of preferably 0.5 to 10 µm, more preferably 1 to 8 µm, and further more preferably 2 to 8 µm.

In the cepharanthine-containing powder, sugar and sugar alcohol can also be used as additives. Here, examples of the sugar include lactose hydrate, sucrose, and glucose. Examples of the sugar alcohol include erythritol, isomalt, lactitol, maltitol, mannitol, sorbitol, and xylitol.

Specific examples of the inhalation powder include a dry power inhaler (hereinafter, abbreviated to DPI). As the device used for the inhalation powder of the present invention, a device that is commonly used as a DPI can be used. For example, examples of the device using capsules include Monohaler, HandiHaler, Breezhaler, and FlowCap. In addition, Diskhaler, Diskus, and Ellipta using aluminum blisters are mentioned.

Examples of a reservoir-type device filled with a powder in a container include Turbuhaler, Clickhaler, Swinghaler, and Twisthaler.

The inhalation liquid is an inhalation preparation in a liquid form that is inhaled with a nebulizer or the like. Cepharanthine may be dissolved or suspended in an appropriate solvent to prepare a solution or suspension. An isotonizing agent, a pH adjuster, and the like can be added during the preparation.

The droplet of the inhalation liquid may be controlled to a droplet of preferably 0.5 to 10 µm, more preferably 1 to 8 µm, and further more preferably 2 to 8 µm according to the configuration of the nebulizer (such as a jet type, an ultrasonic type, and a mesh type), specifically, may be controlled to a droplet having an aerodynamic mass median diameter of preferably 0.5 to 10 µm, more preferably 1 to 8 µm, and further more preferably 2 to 8 µm.

As the cepharanthine according to the inhalation liquid of the present invention, a Cepharanthin (R) injection, which is manufactured and sold by Medisa Shinyaku Inc., can be used. Specifically, a Cepharanthin (R) injection may be inhaled using a nebulizer directly or after dilution with an appropriate solvent.

A device commonly used as a nebulizer can be used as the device used for the inhalation liquid of the present invention. Examples thereof include a type of nebulizing a drug solution with compressed air (a jet type), a type of nebulizing a drug solution using vibration of ultrasonic transducer (a jet type), and a type of nebulizing a drug solution by extruding the drug solution through mesh holes by vibration or the like (a mesh type).

The inhalation aerosol is a metered-dose spray inhalant that facilitates to spray a certain amount of cepharanthine together with a propellant filled in the container.

The spray droplet to be sprayed by the inhalation aerosol may be adjusted to a spray droplet of preferably 0.5 to 10 µm, more preferably 1 to 8 µm, and further more preferably 2 to 8 µm by adjusting the composition of the solution or suspension of cepharanthine, the propellant to be filled, the shape of the nozzle as a member of the container, and the like. Specifically, the spray droplet may be adjusted to a droplet having an aerodynamic mass median diameter of preferably 0.5 to 10 µm, more preferably 1 to 8 µm, and further more preferably 2 to 8 µm.

The inhalation aerosol can be produced by dissolving or suspending cepharanthine in an appropriate solvent to prepare a solution or suspension, filling a pressure proof container with the solution or suspension together with a propellant liquid, and attaching a metering valve thereto. A dispersant, a stabilizer, and the like can be added during the preparation of the solution or suspension.

Example of the inhalation aerosol include a pressurized metered dose inhaler.

The inhalant of the present invention can be used as a SARS-CoV-2 infection inhibitor or a COVID-19 prophylactic and/or therapeutic agent, in addition to alopecia areata/alopecia pityriasis, secretory otitis media, and pit viper bite which are indications of cepharanthine. Although the dosage thereof varies depending on the weight, age, sex, symptoms, and the like of the patient, the dosage for adults is usually in a range of 1 to 20 mg of cepharanthine per day. In addition, when the inhalant of the present invention is used as a SARS-CoV-2 infection inhibitor or a COVID-19 prophylactic and/or therapeutic agent, an anti-HIV agent, such as Nelfinavir, can also be used in combination. Examples

The present invention will now be described specifically by Examples below but the present invention is not limited by these Examples.

### Example 1

Lactose hydrate (98.5 g) was added to cepharanthine (particle diameter: 1.2 µm: measured by a laser diffraction method, 1.5 g) pulverized with a jet mill, followed by mixing with High Flex Gral (manufactured by EARTHTECHNICA Co., Ltd., HF-GS-2J). The resulting powder (0.1 g) was filled in a capsule to prepare an inhalation powder.

### Comparative Example 1

Lactose hydrate (98.5 g) was added to unpulverized cepharanthine (particle diameter: 50 µm: measured by a laser diffraction method, 1.5 g), followed by mixing with High Flex Gral (manufactured by EARTHTECHNICA Co., Ltd., HF-GS-2J). The resulting powder (0.1 g) was filled in a capsule to prepare an inhalation powder.

### Test Example 1

The inhalation powders obtained in Example 1 and Comparative Example 1 were each measured for the Stage 2 expression rate (%) and the fine particle dose (FPD) (%) using Monohaler as a device. The results are shown in Table 1.

### (1) Stage 2 expression rate (%)

The Stage 2 expression rate, which is a rate of reaching the respiratory tract, was determined using Twin Impinger, which is an in vitro evaluation apparatus for inhalants.

### (2) Fine particle dose (FPD) (%)

The evaluation was performed in accordance with the aerodynamic particle size measurement method for inhalants described in The Japanese Pharmacopoeia 17th Edition 2nd Supplement using a multi-stage liquid impinger of the apparatus 1.

**[Table 1]**

| Raw material name | Example 1 | Comparative Example 1 |
|---|---|---|
| Cepharanthine (pulverized) | 1.5 | |
| Cepharanthine (unpulverized) | | 1.5 |
| Lactose | 98.5 | 98.5 |
| TI Stage2 expression rate (%) | 29 | 3 |
| MSLI fine particle dose (FPD) (%) | 29 | 3 |

As is evident from the results of Table 1, in the inhalation powder of Example 1, the Stage 2 expression rate (%) and the fine particle dose (FPD) (%) were each as high as about 30%. It was considered to be possible to deliver cepharanthine to the deep part of the lung.

In contrast, as to unpulverized cepharanthine of Comparative Example 1, the Stage 2 expression rate (%) and the fine particle dose (FPD) (%) were each as low as about 3%. It was considered to be difficult to deliver cepharanthine to the deep part of the lung.

### Example 2

Cepharanthine (1.5 g) was suspended in 100 g of sterile purified water with a stirrer, thereto trometamol (0.1 g) and ethanol (0.1 g) were added and dissolved, and sodium chloride (0.7 g) was added and dissolved to prepare an inhalant.

### Example 3

Cepharanthin (R) injection was used as an inhalation liquid. The composition of Cepharanthin (R) injection 10 mg (2 mL) contains cepharanthine (10 mg) as an active ingredient and benzyl alcohol (40 mg), sodium chloride (20 mg), and hydrochloric acid (appropriate amount) as additives (see the package insert of Cepharanthin (R) injection 10 mg).

### Test Example 2

The inhalation liquids obtained in Examples 2 and 3 were each verified visually whether the agent was sprayed in a mist form using a nebulizer as a device. Furthermore, the agent in a mist form was collected, and the particle diameter thereof was measured with a microscope. The results are shown in Table 2. A liquid sprayed in a mist form is indicated as Good in the Table.

**[Table 2]**

| | Example 2 | Example 3 |
|---|---|---|
| Cepharanthine | 1.5 | 10 |
| Purified water | 100 | 2 |
| Trometamol | 0.1 | |
| Ethanol | 0.1 | |
| Benzyl alcohol | | 40 |
| Hydrochloric acid | | q.s. |
| Sodium chloride | 0.7 | 20 |
| Visual verification (mist form) | Good | Good |
| Particle diameter (µm) | 4 | 5 |

Both the drug solutions of Examples 2 and 3 were sprayed in a mist form, and the particle diameter was about 4 µm in Example 2 and about 5 µm in Example 3. The drug solutions of Examples 2 and 3 were judged to be suitable as inhalation liquids.

## Claims

1. An inhalant comprising cepharanthine.

2. The inhalant according to claim 1, wherein the cepharanthine has a particle diameter of 0.5 to 10 µm.

3. An inhalation powder comprising cepharanthine.

4. The inhalation powder according to claim 3, wherein the cepharanthine has a particle diameter of 0.5 to 10 µm.

5. An inhalation liquid comprising cepharanthine.

6. An inhalant comprising cepharanthine having an aerodynamic mass median diameter of 0.5 to 10 µm.

7. An inhalation powder comprising cepharanthine having an aerodynamic mass median diameter of 0.5 to 10 µm.
